# EUROPEAN PATENT APPLICATION

(11) **EP 1 302 770 A1**
(43) Date of publication of application: **16.04.2003**
(21) Application number: 01948019.3
(22) Date of filing: 13.07.2001
(51) Int. Cl.: G01N 33/543

(54) **PARTICLE-LABELED PROTEIN AND IMMUNO-CHROMATOGRAPH USING THE SAME**

(30) Priority: 14.07.2000 JP 2000214052
(71) Applicant: MATSUSHITA ELECTRIC INDUSTRIAL CO., LTD., Kadoma-shi, Osaka 571-8501 (JP)
(72) Inventor: SHIGETOU, Nobuyuki, Kyotanabe-shi, Kyoto 610-0354 (JP); NAKAYAMA, Hiroshi, Hirakata-shi, Osaka 573-1114 (JP)
(74) Representative: Nachshen, Neil Jacob
(86) International application number: JP0106113
(87) International publication number: WO02006837

(57) **Abstract**

The present invention provides a microparticle and a microparticle-labeled protein using that microparticle for use in chromatography. A protein is bound or adsorbed to the microparticle to form a protein complex, i.e., the microparticle-labeled protein. The microparticle has an absorbance (e.g., at least 0.1 under the measurement conditions: the concentration of the microparticle is 10¹⁰/ml and the optical path length of the microparticle is 1 cm) measurable for a wavelength of about 650 to about 800 nm, preferably about 400 to about 800 nm, and more preferably at least a partial range (e.g., at a portion in the range, and preferably a wavelength range having a width of at least about 50 nm) within the wavelength range of about 200 nm to about 800 nm, and even more preferably all wavelengths in the wavelength range of about 200 nm to about 800 nm.

## Description

### TECHNICAL FIELD

The present invention relates to a microparticle-labeled protein for use in immunological detection, such as an immunoagglutination reaction, immunochromatography, or the like, and an immunochromatography apparatus using the same.

### BACKGROUND ART

Conventionally, a label substance for labeling a protein, which is used in immunological detection, includes a red gold colloid particle, a blue-colored latex particle, and the like. Labeled proteins prepared using these labels are mainly employed in a latex agglutination method, immunochromatography, and the like. However, the blue latex, which has poor visibility for the naked eye, cannot be said to be suitable for blood assays in which a background is red.

Immunochromatography is a measurement method in which the high specificity and detection sensitivity of an antigen-antibodyreactionare utilized, and a substance (e.g., an antibody) capable of specifically reacting with a substance to be measured is used as a detection means. This method is presently widely used as a basic technique for in vitro diagnostics. Among the typical in vitro diagnostics are pregnancy diagnostics, which are immunochromatography apparatuses. A representative structure of the pregnancy diagnostic is disclosed in, for example, US Patent No. 5,602,040. In this immunochromatography apparatus, a gold colloid-labeled antibody is used as a labeled protein, urine is used as a sample to be measured, hCG (subject substance) and the gold colloid-labeled antibody are caused to form a complex, and the presence or absence of a red-colored image derived from the complex is determined with visual observation.

The above-described conventional labeled protein is labeled with a microparticle which does not absorb light having a specific wavelength region, i.e., 400 nm to 800 nm, about 600 to 800 nm, and particularly 650 to 800 nm within wavelength 200 to 800 nm. Therefore, in order to perform immunological detection using a colored sample, a microparticle having a color different from that of the sample, i.e., not having a wavelength region different from that of the sample, has to be used. Therefore, it was necessary to prepare a different labeled protein depending on the color of a sample. Specifically, for example, when it is attempted to immunologically detect a trace amount of marker or the like contained in blood, use of a red gold colloid-labeled antibody as a labeled protein makes it difficult to determine the color with visual observation. In this case, it is difficult to perform high-precision measurement. On the other hand, blue latex has poor visibility and is not therefore useful.

An object of the present invention is to give a solution to the above-described problems by providing a microparticle and a microparticle-labeled protein produced with that microparticle, and an immunochromatography method and apparatus, which are capable of measuring in a simple and highly precise manner a sample which is conventionally difficult to measure in such a manner. Another object of the present invention is to provide a microparticle and a microparticle-labeled protein produced with that microparticle, and an immunochromatography method and apparatus, which are capable of performing qualitative measurement and quantitative measurement using immunological detection, such as immunochromatography or the like, in a simple and highly precise manner without depending on the color of a sample.

### DISCLOSURE OF THE INVENTION

In order to solve the above-described problems, the present invention provides a microparticle and a microparticle-labeled protein using that microparticle for use in chromatography. A protein is bound or adsorbed to the microparticle to form a protein complex, i.e., the microparticle-labeled protein. The microparticle has an absorbance (e.g., at least 0.1 under the measurement conditions: the concentration of the microparticle is 10¹⁰/ml and the optical path length of the microparticle is 1 cm) measurable for a wavelength of about 650 to about 800 nm, preferably about 400 to about 800 nm, and more preferably at least a partial range (e.g., at a portion in the range, and preferably a wavelength range having a width of at least about 50 nm) within the wavelength range of about 200 nm to about 800 nm, and even more preferably all wavelengths in the wavelength range of about 200 nm to about 800 nm. The microparticle may usually have a grain diameter of at least 10 nm and no more than 10 µm.

In one embodiment, the microparticle may have a measurable absorbance over a range having a width of at least about 50 nm, preferably 100 nm, and more preferably 150 nm within the above-described wavelength range. If these wavelength ranges having the measurable absorbance exceed about 150 nm (e.g., about 400 nm, about 600 nm, etc.), these wavelength ranges may be about 200 nm, about 250 nm, about 300 nm, about 350 nm, about 400 nm, about 450 nm, about 500 nm, about 550 nm, about 600 nm, or the like.

In another embodiment, the microparticle has a measurable absorbance over a certain range at two or more (e.g., 3, 4, or the like) different positions within the wavelength range. The certain range preferably has a width of about 50 nm, more preferably at least about 100 nm, more preferably about 200 nm, about 250 nm, about 300 nm, about 350 nm, about 400 nm, or the like.

In a more preferable embodiment, the microparticle may have a measurable absorbance for all wavelengths in the above-described wavelength range.

In one embodiment, the measurable absorbance may be at least about 0.1 under conditions that the concentration of the microparticle is at least 10¹⁰/ml and the optical path length of the microparticle is 1 cm. Preferably, this absorbance may be at least about 0.2 under these conditions. More preferably, the absorbance may be at least about 0.3, at least about 0.4, at least about 0.5, at least about 0.75, at least about 1.0, or the like.

In one embodiment, the above-described wavelength range may be about 600 to about 800 nm, and more preferably about 400 to about 800 nm. More preferably, the wavelength range may be about 200 to about 800 nm. The upper and lower limits of the wavelength range can be arbitrarily determined depending on a subject substance, measurement conditions, or the like. The lower limit may be less than 200 nm, and the upper limit may exceed 800 nm. Examples of the lower limit include about 100 nm, about 110 nm, about 120 nm, about 130 nm, about 140 nm, about 150 nm, about 160 nm, about 170 nm, about 180 nm, about 190 nm, about 200 nm, about 210 nm, about 220 nm, about 230 nm, about 240 nm, about 250 nm, about 260 nm, about 270 nm, about 280 nm, about 290 nm, about 300 nm, about 310 nm, about 320 nm, about 330 nm, about 340 nm, about 350 nm, about 360 nm, about 370 nm, about 380 nm, about 390 nm, about 400 nm, about 410 nm, about 420 nm, about 430 nm, about 440 nm, about 450 nm, about 460 nm, about 470 nm, about 480 nm, about 490 nm, about 500 nm, about 510 nm, about 520 nm, about 530 nm, about 540 nm, about 550 nm, about 560 nm, about 570 nm, about 580 nm, about 590 nm, about 600 nm, about 610 nm, about 620 nm, about 630 nm, about 640 nm, about 650 nm, about 660 nm, about 670 nm, about 680 nm, about 690 nm, about 700 nm, about 710 nm, about 720 nm, about 730 nm, about 740 nm, about 750 nm, about 760 nm, about 770 nm, about 780 nm, about 790 nm, and the like. On the other hand, examples of the upper limit include about 110 nm, about 120 nm, about 130 nm, about 140 nm, about 150 nm, about 160 nm, about 170 nm, about 180 nm, about 190 nm, about 200 nm, about 210 nm, about 220 nm, about 230 nm, about 240 nm, about 250 nm, about 260 nm, about 270 nm, about 280 nm, about 290 nm, about 300 nm, about 310 nm, about 320 nm, about 330 nm, about 340 nm, about 350 nm, about 360 nm, about 370 nm, about 380 nm, about 390 nm, about 400 nm, about 410 nm, about 420 nm, about 430 nm, about 440 nm, about 450 nm, about 460 nm, about 470 nm, about 480 nm, about 490 nm, about 500 nm, about 510 nm, about 520 nm, about 530 nm, about 540 nm, about 550 nm, about 560 nm, about 570 nm, about 580 nm, about 590 nm, about 600 nm, about 610 nm, about 620 nm, about 630 nm, about 640 nm, about 650 nm, about 660 nm, about 670 nm, about 680 nm, about 690 nm, about 700 nm, about 710 nm, about 720 nm, about 730 nm, about 740 nm, about 750 nm, about 760 nm, about 770 nm, about 780 nm, about 790 nm, about 800 nm, about 810 nm, about 820 nm, about 830 nm, about 840 nm, about 850 nm, about 860 nm, about 870 nm, about 880 nm, about 890 nm, about 900 nm, about 910 nm, about 920 nm, about 930 nm, about 940 nm, about 950 nm, about 960 nm, about 970 nm, about 980 nm, about 990 nm, and the like.

In a preferred embodiment, the microparticle of the present invention may be black. In another embodiment, the microparticle may be made of graphite or ferrite.

The microparticle of the present invention may have any grain diameter as long as it can be used in chromatography. The grain diameter may be usually in the range of about 10 nm to about 10 µm.

In another aspect, the present invention provides a microparticle-labeled protein in which the microparticle of the present invention is bound or adsorbed to a protein.

In one embodiment, the protein may be bound or adsorbed to a surface of the microparticle.

In another embodiment of the microparticle-labeled protein, the microparticle maybe made of graphite or ferrite.

In a preferred embodiment, the protein may be a protein which may specifically bind to a certain target. More preferably, this protein is an antibody. Such an antibody may be a monoclonal antibody or a polyclonal antibody.

In one embodiment, a measurable absorbance of the microparticle-labeled protein may be such that a ratio of a reflective absorbance for about 540 nm to a reflective absorbance for about 650 nm is no more than about 2:1, under conditions that the concentration of the microparticle-labeled protein is at least 10¹⁰/ml and an optical path length of the microparticle-labeled protein is 1 cm.

In one embodiment, the microparticle-labeled protein may have substantially the same absorbance as that of the microparticle bound thereto.

In one embodiment, the ratio is in the range of about 2:1 to about 1:2.

In another aspect, the present invention provides a chromatography method of detecting a target substance in a sample. The method may comprise the steps of:
a) producing a microparticle-labeled protein by causing a protein capable of reacting with the target substance to be bound or adsorbed to a microparticle according to any one of claim 1 to 10; and
b) detecting a signal derived from the microparticle by contacting the microparticle-labeled protein with the target substance.

In one embodiment, the target substance is an antigen and the protein is an antibody specific to the antigen.

In another embodiment, the detecting step may be performed by the naked eye. In another embodiment, the detecting step may be performed by spectrophotometry.

In another aspect, the present invention provides a chromatography apparatus using the microparticle or microparticle-labeled protein of the present invention. The chromatography apparatus detects a target substance in a sample based on an antigen-antibody reaction. The chromatography comprises an introducing section for introducing the sample, a labeling section containing the microparticle-labeled protein, and a detecting section in which an antibody capable of binding to an antigen to be detected is immobilized. Preferably, the sample introducing section, the labeling section, and the detecting section may be arranged so that at least a portion of the sample introduced to the sample introducing section is transferred via the labeling section to the detecting section.

In one embodiment, a chromatography apparatus for detecting a target substance in a sample according to the present invention may comprise:
a) an introducing section for introducing the sample;
b) a labeling section containing a microparticle-labeled protein according to any one of claims 11 to 16, wherein the protein is specific to the target substance; and
c) a detecting section for detecting interaction between the target substance and the microparticle-labeled protein.

In one embodiment, the target substance may be an antigen and the protein may be an antibody specific to the antigen.

In another embodiment, the sample introducing section, the labeling section, and the detecting section may be arranged so that at least a portion of the sample introduced to the sample introducing section is transferred via the labeling section to the detecting section.

### BRIEF DESCRIPTION OF THE DRAWINGS

Figure **1** is a graph showing the absorbance spectra of microparticles used in microparticle-labeled proteins according to examples of the present invention and a conventional example.
Figure **2** is a cross-sectional view showing an immunochromatography apparatus according to an example of the present invention.
Figure **3** is a plan view showing an immunochromatography apparatus according to an example of the present invention.

### (Description of Reference Numerals)

In the above-described figures, the following reference numerals indicate what are described on the right side.
**1** sample introducing section
**2** labeling section
**3** detecting section
**4** solution absorbing section
**5** backing sheet

### BEST MODE FOR CARRYING OUT THE INVENTION

Hereinafter, the present invention will be described by way of illustrative examples with reference to the accompanying drawings.

It should be understood throughout the present specification that articles for singular forms (e.g., "a", "an", "the", etc. in English; "ein", "der", "das", "die", etc. and their inflections in German; and articles, adjectives, etc. in other languages) include the concept of their plurality unless otherwise mentioned. It should be also understood that terms as used herein have definitions ordinarily used in the art unless otherwise mentioned.

The present invention provides a microparticle and a microparticle-labeled protein using that microparticle, which are used in chromatography. A protein is bound or adsorbed to a surface of the microparticle to form a microparticle-labeled protein, i.e., a protein complex. In this case, the microparticle has an absorbance (e.g., at least 0.1 under the measurement conditions: the concentration of the microparticle is at least 10¹⁰/ml and the optical path length of the microparticle is 1 cm) measurable for a wavelength of about 650 to about 800 nm, preferably about 400 to about 800 nm, and more preferably at least a partial range (e.g., at a portion in the range, and preferably the wavelength range having a width of at least about 50 nm) within the wavelength range of about 200 nm to about 800 nm, and even more preferably all wavelengths in the wavelength range of about 200 nm to about 800 nm. The microparticle may usually has a grain diameter of at least 10 nm and no more than 10 µm.

Such a microparticle-labeled protein has an absorbance wavelength of 650 nm to 800 nm (preferably exhibits a black color). Therefore, the microparticle-labeled protein can be used in specific detection, such as a chromatography means (e.g., immunochromatography), to easily detect a sample (e.g., a sample which does not have an absorbance wavelength of 650 nm to 800 nm) which is conventionally difficult to detect. In a preferred embodiment of the present invention, since a coloring can be detected with visual observation irrespective of the color of a sample, it is not necessary to prepare a different labeled protein depending on the color of the sample. Therefore, it is possible to perform simple and high-precision qualitative measurement. Moreover, by determining coloring due to an antigen-antibody reaction by measuring the absorbance of a sample using light having a wavelength different from the absorbance wavelength, it is possible to perform simple and high-precision quantitative measurement.

The term "microparticle" refers to a solid substance having a small size with in a certain range (e.g., usually, its grain diameter is at least 10 nm and no more than 10 µm). A protein can be bound or adsorbed to the surface of a microparticle of the present invention. Therefore, the microparticle can be used in chromatography. The microparticle of the present invention has an absorbance (e.g., at least 0.1 under the measurement conditions: the concentration of the microparticle is 10¹⁰/ml and the optical path length of the microparticle is 1 cm) measurable for a wavelength of about 650 to about 800 nm, preferably about 400 to about 800 nm, and more preferably at least a partial range (e.g., at at least a portion in the range, and preferably a wavelength range having a width of at least about 50 nm) within the wavelength range of about 200 nm to about 800 nm, and even more preferably all wavelengths in the wavelength range of about 200 nm to about 800 nm. Examples of the microparticle of the present invention include graphite, ferrite, manganese dioxide (MnO₂), chromium oxide (Cr₂O₃), copper sulfide (CuS), zinc sulfide (ZnS), and silver sulfide (Ag₂S). In this case, the microparticle can be preferably formed in water in the state of stable colloid or in the state of stable suspension and dispersion. Further, the microparticle is preferably graphite or ferrite since they have excellent ability to be bound or adsorbed to a protein. The present invention is not so limited.

The term "protein" has a meaning commonly used in the art, referring to a polymer in which at least two, preferably at least 10, and more preferably at least 50 (native or non-native) amino acids are polymerized with peptide bonds.

It is necessary that the protein has ability to specifically react with a substance to be measured (target substance) in detection based on a specific interaction, and preferably immunologically detection. A conventionally known enzyme and antibody can be used without particular limitation. Such a protein can be easily recognized by those skilled in the art. Preferably, the protein is an antibody.

The term "microparticle-labeled protein" refers to a protein to which a microparticle of the present invention is bound or attached. This microparticle-labeled protein has substantially the same absorbance as that of the original microparticle. Therefore, the microparticle has an absorbance within the above-described wavelength range. Note that an absorbance derived from the protein to which the microparticle is bound may also be observed in addition to the absorbance by the microparticle.

A microparticle-labeled protein of the present invention can be prepared by a method known in the art. For example, the microparticle-labeled protein can be prepared by adding a buffer solution containing an antibody to a suspension solution containing the microparticle and allowing the mixture to stand.

A method known in the art can be applied as a chromatography method for detecting a target substance in a sample. This method may comprise the steps:
a) producing a microparticle-labeled protein by causing a microparticle of the present invention to be bound or absorbed to a protein capable of specifically reacting with the target substance; and
b) detecting a signal derived from the microparticle by contacting the microparticle-labeled protein with the target substance.

The microparticle-labeled protein of the present invention can be prepared using the microparticle of the present invention and a protein by any of various methods as known herein. This preparation may be carried out by an apparatus or a manual operation. Further, this preparation may be incorporated into a chromatography apparatus or may be carried out by a separate apparatus. The apparatus may be an automated apparatus.

The above-described target substance is any substance as long as it has specificity to a certain substance to some extent, and preferably may be an antigen. The above-described protein is any substance as long as it is specific to this target substance, and preferably may be an antibody. The above-described protein is preferably specific to the microparticle of the present invention.

The above-described detecting step may be detection with the naked eye or an apparatus. Examples of detection using an apparatus include spectrophotometry, ultraviolet absorbance measurement, infrared absorbance measurement, X-ray measurement, fluorescence intensity measurement, and turbidimetry.

A chromatography apparatus of the present invention detects a target substance (e.g., an antigen) in a sample based on a specific protein interaction, and preferably an antigen-antibody reaction. This chromatography apparatus may comprise a sample introducing section through which the sample is introduced, a labeling section containing the above-described microparticle-labeled protein in which, for example, an antibody is used as the protein, and a detecting section in which an antibody capable of binding to a substance to be detected (e.g., an antigen) is immobilized. The sample introducing section, the labeling section, and the detecting section are arranged so that at least a part of the sample introduced into the sample introducing section is transferred via the labeling section to the detecting section.

With this configuration, when the sample introduced into the sample introducing section reaches the labeling section, the microparticle-labeled protein contained in the labeling section specifically binds to the antigen contained in the sample. Thereafter, this microparticle-labeled protein is transferred together with the sample to the detecting section. The microparticle-labeled protein binds to the antibody immobilized in the detecting section via the antigen. As a result, the detecting section is colored by the color, preferably black, which is processed by the microparticle of the present invention. Therefore, even when a colored sample is used, the coloring different from that of the sample can be determined with visual observation. Preferably, detection can be carried out with visual observation irrespective of the color of a sample. It is thus possible to perform simple and high-precision qualitative measurement without preparing a different labeled protein depending on the color of a sample. Further, by determining the coloring in the detecting section by absorbance measurement using light having a wavelength different from that of the sample, it is possible to perform simple and high-precision quantitative measurement.

The above-described sample is not particularly limited and any sample can be used. A particular advantageous effect is obtained when a biological sample, such as blood, urine, saliva, stool, or the like, is used.

The above-described sample introducing section, labeling section and detecting section can be made of a porous carrier, such as glass filter, nitrocellulose, or the like, which is a conventionally known material.

The above-described labeling section and detecting section can be prepared by a conventionally known method. For example, the labeling section can be prepared by impregnating a porous carrier with a microparticle-labeled protein, followed by lyophilization. The detecting section can be prepared by dropping an aqueous antibody solution onto a porous carrier, in any form, followed by drying and washing.

### Examples

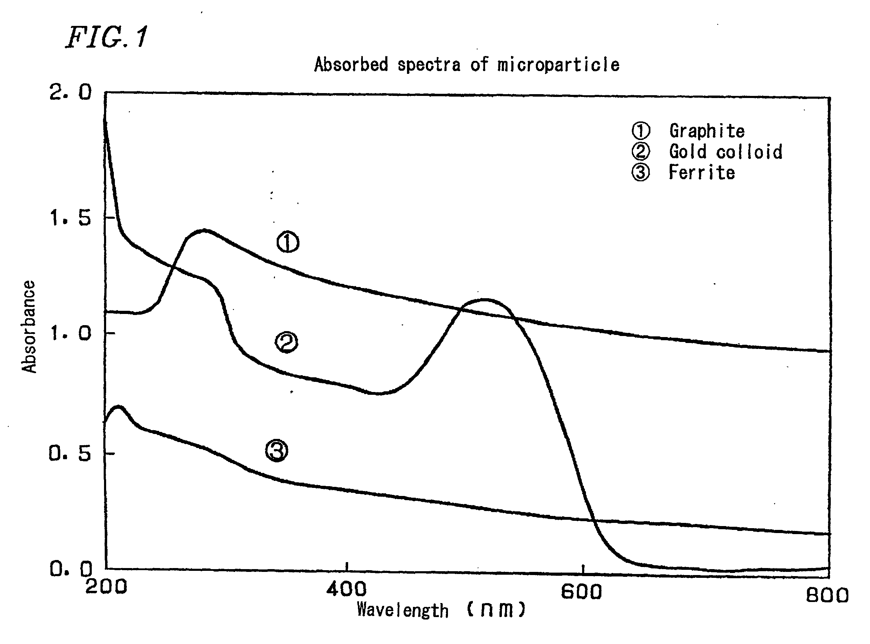

Hereinafter, examples of the present invention will be described with reference to Figures **1** to **3**. It should be understood that the examples below are only intended to illustrate the present invention, but are not intended to limit the present invention.

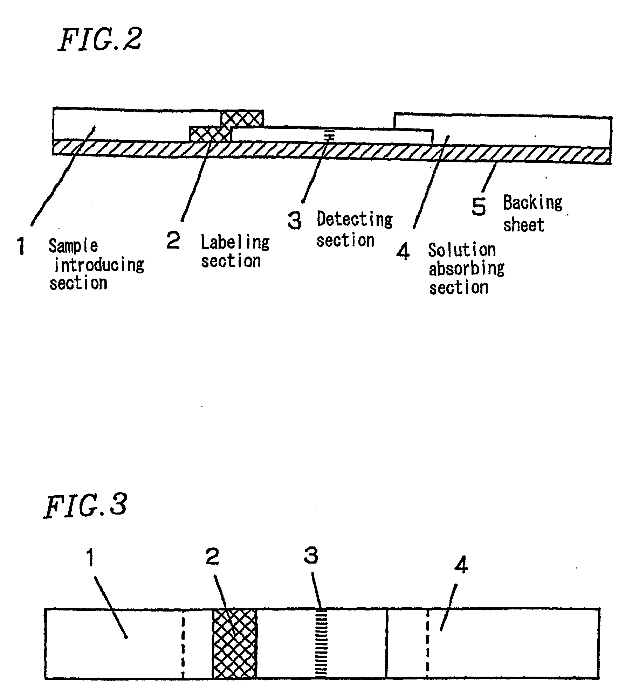

Figure **1** is a graph showing the absorbance spectra of microparticles used in microparticle-labeled proteins according to an example of the present invention and a conventional example. Figure **2** is a cross-sectional view showing an immunochromatography apparatus according to the present invention. Figure **3** is a plan view showing an immunochromatography apparatus according to the present invention. Note that in the examples, an anti-C reactive protein (hereinafter abbreviated as CRP) monoclonal antibody is used as a protein, immunochromatography is used as a method using a microparticle-labeled protein, and CRP is used as a substance to be detected.

### (Example 1)

In Example 1, the microparticle used was made of ferrite, which had a diameter of about 15 nm. This ferrite particle was used to prepare a suspension solution having a concentration of 3.792×10¹²/ml, which was in turn adjusted to pH=9 using 0.2 M sodium carbonate aqueous solution. 300 µg of an anti-CRP monoclonal antibody was added to 5 ml of the suspension solution while mildly stirring. Stirring was continued at room temperature for 5 minutes. Thereafter, the suspension solution was centrifuged at about 40,000 G. After removal of the supernatant, about 5 ml of phosphate buffer solution (pH=7.5, hereinafter abbreviated as PBS) was added to the precipitate so that the precipitate was dispersed again. The resultant suspension solution was centrifuged at about 8,000 G, followed by removal of the supernatant. Thus, a ferrite-labeled anti-CRP monoclonal antibody was obtained.

### (Example 2)

In Example 2, the microparticle used was made of graphite, which had a diameter of about 80 nm. This graphite particle was used to prepare a suspension solution having a concentration of 1×10¹¹/ml, which was in turn adjusted to pH=9 using 0.2 M sodium carbonate aqueous solution. 300 µg of an anti-CRP monoclonal antibody was added to 5 ml of the suspension solution while mildly stirring. Stirring was continued at room temperature for 5 minutes. Thereafter, the suspension solution was centrifuged at about 8,000 G. After removal of the supernatant, about 5 ml of pure water was added to the precipitate so that the precipitate was dispersed again. The resultant suspension solution was centrifuged at about 8,000 G, followed by removal of the supernatant. Thus, a graphite-labeled anti-CRP monoclonal antibody was obtained.

### (Comparative Example 1)

As Comparative Example 1, an antibody labeled with a gold colloid having a grain diameter of about 20 nm as a microparticle was prepared as follows. This gold colloid was used to prepare a colloid solution having a concentration of 10¹²/ml, which was in turn adjusted to pH=9 using 0.2 M sodium carbonate aqueous solution. 330 µg of an anti-CRP monoclonal antibody was added to this colloid solution while mildly stirring. Stirring was continued at room temperature for 5 minutes. Thereafter, 5.5 ml of 10% bovine serum albumin (hereinafter abbreviated as BSA) aqueous solution was added to the suspension solution, followed by stirring for two minutes. The resultant reaction solution was centrifuged at about 40,000 G for one hour while being maintained at 4°C, followed by removal of the supernatant. 0.1 M Tris · 1% BSA solution (pH=8.2) was added to the precipitate so that the precipitate was dispersed again. Thereafter, the resultant suspension was centrifugedat about 8,000 G for one hour while being maintained at 4°C. The supernatant was removed. Thus, a gold colloid-labeled anti-CRP monoclonal antibody was removed.

### (Measurement of the Absorbance Spectrum of a Microparticle)

The absorbance spectra of the microparticles used in Example 1, Example 2 and Comparative Example 1 were measured. For measurement, a UVPC-1600 spectrometer (manufactured by Hitachi Ltd.) was used. As can be seen from Figure **1**, whereas the ferrite microparticle used in Example 1 and the graphite microparticle used in Example 2 absorbs light having any wavelength in the entire range of 200 nm to 800 nm, the gold colloid microparticle does not absorb light having a wavelength of about 650 nm to 800 nm.

### (Preparation of an Immunochromatography Apparatus and Measurement of an Antigen using the same)

Glass filters were impregnated with the ferrite-labeled anti-CRP monoclonal antibody of Example 1 and the graphite-labeled anti-CRP monoclonal antibody in Example 2, and the gold colloid-labeled anti-CRP monoclonal antibody of Comparative Example 1, followed by lyophilization. This lyophilized glass filter was used as a labeling section to prepare an immunochromatography apparatus having the structure shown in Figures **2** and **3.** A solution absorbing section **4** (0.9 cm x 2 cm) made of glass fiber filter was adhered to a surface of a backing sheet **5** (0.9 cm × 5.5 cm) made of rigid polyvinyl chloride having double-sided adhesive tape on the entire surface. A detecting section **3** (0.9 cm × 2 cm) made of nitrocellulose membrane, on which an anti-CRP monoclonal antibody was immobilized, was adhered to the backing sheet **5** at a position upstream of the solution absorbing section **4**. A labeling section **2** (0.9 cm × 1 cm) impregnated with the labeled anti-CRP monoclonal antibody of Example 1, Example 2 or Comparative Example 1 was adhered to the backing sheet **5** at a position upstream of the detecting section **3**. A sample introducing section **1** (0.9 cm × 2.5 cm) made of glass fiber filter was adhered to the backing sheet 5 at a position upstream of the labeling section **2**. The resultant structure was covered with cellophane tape (not shown), leaving a part of the transparent sample introducing section **1** uncovered. The thus-obtained immunochromatography apparatus was incorporated into a hollow housing (not shown) and was used for measurement.

0.1 mg/dl of water containing CRP was added as a sample to the sample introducing section of the prepared immunochromatography apparatus. After 10 minutes, the level of coloring in the detecting section was measured as a reflective absorbance at 540 nm and 650 nm. The result is shown in Table 1.

**Table 1**

| | Microparticle | Reflective absorbance | |
|---|---|---|---|
| | | 540 nm | 650 nm |
| Example 1 | Ferrite | 0.320 | 0.230 |
| Example 2 | Graphite | 0.830 | 0.655 |
| Comparative Example 1 | Gold colloid | 0.665 | 0.225 |

As can be seen from Table 1, in the case of any of the above-described examples, the reflective absorbance at 540 nm was reduced from the reflective absorbance at 650 nm, though the reduction rate was smaller for the immunochromatography apparatus using the microparticle-labeled protein of Example 1 and Example 2 than for the immunochromatography apparatus using the microparticle-labeled protein of Comparative Example 1.

For example, a red color blood sample has a high absorbance around 500 to 600 nm, but has substantially no absorbance in the wavelength region of at least 600 nm, and particularly at least 650 nm. Specifically, the red color blood sample has an absorbance of no more than 0.1 under the following conditions: the concentration of the microparticle is at least 10¹⁰/ml and the optical path length of the microparticle is 1 cm. Therefore, when the sample is blood, it is difficult to perform qualitative measurement with visual observation in the immunochromatography apparatus using a microparticle-labeled protein of Comparative Example 1 since the color in the detecting section is red. As can be seen in Figure **1** and Table 1, it is also difficult to perform quantitative measurement even when light having a particular wavelength is used for absorbance measurement, since the absorbance is steeply reduced in the range of at least about 600 nm. In contrast, in the immunochromatography apparatus using a microparticle-labeled protein of Example 1 or Example 2, the color in the detecting section is black, thereby making it easy to perform qualitative measurement. Further, as can be seen from Figure **1** and Table 1, the microparticle has an absorbance for at least 600 nm. Therefore, it is possible to perform quantitative measurement by measuring absorbance using light having a particular wavelength of at least 600 nm.

Note that in the above-described Examples, the ferrite microparticle having a grain diameter of about 15 nm and the graphite microparticle having a grain diameter of about 80 nm were used as microparticles. The present invention is not so limited. A microparticle having a grain diameter in the range of 10 nm to 10 µm can be used to obtain the same effect.

### INDUSTRIAL APPLICABILITY

As described above, according to the present invention, it is possible to easily detect a color based on an immunological reaction irrespective of the color of a sample. Therefore, the microparticle-labeled protein of the present invention can be used to perform qualitative and quantitative measurement in immunological detection, such as immunochromatography or the like, in a simple and high-precision manner. Further, the immunochromatography apparatus of the present invention can be used to perform qualitative and quantitative measurement in immunochromatography in a simple and high-precision manner.

## Claims

1. A microparticle for use in chromatography, wherein the microparticle has a measurable absorbance for at least a part of a wavelength range of about 650 to about 800 nm.

2. A microparticle according to claim 1, wherein the microparticle has a measurable absorbance over a range having a width of at least about 50 nm within the wavelength range.

3. A microparticle according to claim 1, wherein the microparticle has a measurable absorbance over a range having a width of at least about 50 nm at two or more different positions within the wavelength range.

4. A microparticle according to claim 1, wherein the microparticle has a measurable absorbance over all wavelengths in the wavelength range.

5. A microparticle according to any one of claims 1 to 4, wherein the measurable absorbance is at least 0.1 under measurement conditions such that a concentration of the microparticle is at least 10¹⁰/ml and an optical path length of the microparticle is 1 cm.

6. A microparticle according to claim 5, wherein the measurable absorbance is at least about 0.2 under the conditions.

7. A microparticle according to any one of claims 1 to 6, wherein the wavelength range is about 400 to about 800 nm.

8. A microparticle according to any one of claims 1 to 6, wherein the wavelength range is about 200 to about 800 nm.

9. A microparticle according to claim 1, wherein the microparticle is black.

10. A microparticle according to any one of claims 1 to 9, wherein the microparticle has a grain diameter of about 10 nm to about 10 µm.

11. A microparticle-labeled protein, wherein a microparticle according to any one of claims 1 to 10 is bound or adsorbed to a protein.

12. A microparticle-labeled protein according to claim 11, wherein the protein is bound or absorbed to a surface of the microparticle.

13. A microparticle-labeled protein according to claim 11 or 12, wherein the microparticle is made of graphite or ferrite.

14. A microparticle-labeled protein according to any one of claims 11 to 13, wherein the protein is an antibody.

15. A microparticle-labeled protein according to any one of claims 11 to 14, wherein a measurable absorbance of the microparticle-labeled protein is such that a ratio of a reflective absorbance for about 540 nm to a reflective absorbance for about 650 nm is no more than about 2:1, under conditions that a concentration of the microparticle-labeled protein is at least 10¹⁰/ml and an optical path length of the microparticle-labeled protein is 1 cm.

16. A microparticle-labeled protein according to claim 15, wherein the ratio is in the range of about 2:1 to about 1:2.

17. A chromatography method of detecting a target substance in a sample, comprising the steps of:
a) producing a microparticle-labeled protein by causing a protein capable of reacting with the target substance to be bound or adsorbed to amicroparticle according to any one of claim 1 to 10; and
b) detecting a signal derived from the microparticle by contacting the microparticle-labeled protein with the target substance.

18. A method according to claim 17, wherein the target substance is an antigen and the protein is an antibody specific to the antigen.

19. A method according to claim 17 or 18, wherein the detecting step is performed by the naked eye.

20. A chromatography apparatus for detecting a target substance in a sample, comprising:
a) an introducing section for introducing the sample;
b) a labeling section containing a microparticle-labeled protein according to any one of claims 11 to 16, wherein the protein is specific to the target substance; and
c) a detecting section for detecting interaction between the target substance and the microparticle-labeled protein.

21. A chromatography apparatus according to claim 20, the target substance is an antigen and the protein is an antibody specific to the antigen.

22. A chromatography apparatus according to claim 20 or 21, wherein the sample introducing section, the labeling section, and the detecting section are arranged so that at least a portion of the sample introduced to the sample introducing section is transferred via the labeling section to the detecting section.
